# EUROPEAN PATENT SPECIFICATION

(11) **EP 1 237 604 B1**
(45) Date of publication and mention of the grant of the patent: **28.12.2005**
(21) Application number: 00971531.9
(22) Date of filing: 20.10.2000
(51) Int. Cl.: A61M 15/00, A61J 1/03, A61J 3/00

(54) **DOSAGE UNIT FOR DRY POWDER MEDICAMENT**
DOSIERBEHÄLTER FÜR PULVERFÖRMIGE MEDIKAMENTE
UNITE POSOLOGIQUE POUR MEDICAMENT EN POUDRE SECHE

(30) Priority: 22.10.1999 GB 9924950; 08.12.1999 GB 9929025
(43) Date of publication of application: 11.09.2002
(73) Proprietor: Innovata Biomed Limited, St. Albans AL1 3HW (GB)
(72) Inventor: BRAITHWAITE, Philip, ML Laboratories Plc, Tewkesbury GL20 8NB (GB); WILLIAMS, Steven Graham, ML Laboratories Plc, Tewkesbury GL20 8NB (GB)
(74) Representative: Jones, Stephen Anthony
(86) International application number: PCT/GB2000/004042
(87) International publication number: WO 2001/030430

(56) References cited:
- WO-A-93/16748
- US-A- 3 439 823
- US-A- 5 411 175
- US-A- 5 562 918

## Description

This invention relates to a novel form of drug container and to medical devices utilising such containers.

International Patent Application No. WO 93/16748 describes an inhalation device and in particular a dry powder inhaler, known as TECHNOHALER being developed by Innovata Biomed in the UK. The TECHNOHALER is provided with a plurality of capsules, usually in the form of a cartridge, wherein each capsule contains a single metered dose of medicament. The capsule of the prior art generally is made up of a spool and a spool carrier. The spool comprises a longitudinal body and terminal flanges at each end. The sides of the flanges form a seal and a tight slidable fit with the inner walls of the spool carrier. The length of the spool and the length of the spool carrier are substantially the same and medicament fills the space between the spool and the internal walls of the spool carrier.

Furthermore a dry powder inhaler such as TECHNOHALER is provided with suitable indexing means, including a push button and a ratchet mechanism which engages with the upper surface of the container. In TECHNOHALER depression of the push button urges a push rod to act against the spool which is nearest to the inhalation passage of the inhaler and into the inhalation passage.

The spool is pressed out of the spool carrier releasing medicament. The spool is not fully ejected from the spool carrier so that upon rotation of the cartridge the spool is transferred to a waste chamber. Nevertheless, in order to ensure all of the medicament in the capsule is ejected into the inhalation passage the spool must undergo considerable displacement by being pushed close to being ejected from the carriers.

US Patent No. 5,411,175 describes a cartridge for providing liquid to be dispensed. The cartridge comprises a liquid holding chamber and a sleeve which extends beyond the length of the holding chamber, the unit being provided with an openable closure member.

We have now found a novel medicament metered dosage unit which is advantageous in that, inter alia, only a minimal amount of displacement is required for the dispensing of the medicament.

Thus according to the invention we provide a medicament dosage unit comprising a sleeve and a medicament holding chamber adapted to form a slidable fit within the sleeve, the sleeve and the chamber being dimensioned such that the sleeve extends beyond the length of the chamber, and the unit being provided with an openable closure member.

The dimensions of the medicament chamber may be varied, permitting different dosages of medicament to be administered. Preferably, the dimensions are such that the chamber will be filled to provide a single desired dose. In one embodiment the chamber is a substantially elongate member eg a cylindrical member with an open end and a closed end. When the closure member comprises a removable cap, the cap may rest on the sleeve. However, preferentially, the chamber may be provided with one or more spacers at its open end. Preferably at least two spacers are present to allow even resting of the cap. The use of spacers is advantageous in that they prevent the cap from coming into contact with the medicament and possibly reducing the accuracy of the dosage delivered. The spacers can also act to enhance removal of the cap. The spacers may optionally be provided with a ridge upon which the cap may rest.

The cap may generally be the same diameter as the medicament chamber. The cap may comprise a flat disc, a plug or an inverted cup. It is desirable that the cap should provide a closed face abutting the chamber and/or spacers. The length of the cap will be small relative to the length of the chamber.

The sleeve is adapted to form a snug fit at least around the joint formed between the open end of the container and the cap. The sleeve preferentially wraps around the whole of the circumference of the joint so as to form a seal. The sleeve comprises a substantially resilient material, e.g. a plastics sleeve, in order for the inner walls of the sleeve to be biased towards the joint so as to form a sealing engagement. In a preferred embodiment a longitudinal sleeve is used enabling it to also act as a support for the body of the chamber. Thus, it is preferred that the length of the sleeve will be substantially the same as the length of the chamber. In an especially preferred embodiment the length of the sleeve is such that it forms a snug fit with the chamber with only the spacers protruding from the sleeve. When the cap is placed upon the spacers and urged against the chamber so that the container is partially pushed through the sleeve, the closed end of the container protrudes from the sleeve and the sleeve forms a sealing engagement with the cap and the container.

When the closure member comprises a cover, the cover is preferably fixed to the sleeve. The cover will generally be the same diameter as the sleeve or, optionally, it may be of slightly greater dimensions such that it overlaps the end of the sleeve. The cover may preferentially comprise any frangible material. Materials which are impermeable to moisture and/or are moisture resistant are preferred. Such materials include, but are not limited to, plastics films or foils, e.g. aluminium foil material. In the case of a plastics cover, this may simply be heat bonded to the sleeve, whilst with a foil cover, a layer of conventionally used adhesive may be used to bond the foil to the sleeve.

The dosage unit may also be one of a plurality of such units arranged in series, which units are able to transfer a succession of metered doses of medicament into the inhalation passage of a dry powder inhaler. When a plurality of dosage units are connected together, the sleeves required may be comprised of a cartridge with a plurality of sleeves arranged around its periphery. In such a case the dosage units themselves may be connected together or it may be that the sleeves are connected together, or both.

The invention thus also provides a plurality of dosage units arranged in series, each unit being as hereinbefore described. The units may be releasably or permanently attached to one another so as to be in a chain-like conformation, preferably a flexible or semi-flexible chain. The design of dosage units in accordance with the invention makes such flexibility possible.

A series of dosage units in accordance with this aspect of the invention is ideal for use in an inhaler, because it allows sequential presentation of doses of a medicament to the inhalation passage of the inhaler as the series is indexed through the inhaler. If the series is in the form of a flexible chain, it can then be rolled or folded up for compact storage in the inhaler. The series may be of any appropriate length. It may, for instance, be supplied in a length greater than might be needed for use in an inhaler, but capable of being broken up into usable lengths. In an especially preferred embodiment the plurality of dosage units are contained in a cartridge and such a cartridge forms a further aspect of the invention.

In use, when placed in an inhaler, such as the TECHNOHALER, a push rod can act upon the closed end of the container protruding from the sleeve, urging the container back in the sleeve, and causing the cap to be ejected from the other end of the sleeve. When the container is in the inverted position, that is, the closed end uppermost, the cap falls away and the container empties the medicament into the inhalation passage of the inhaler.

Thus, according to a further feature of the invention we provide a medicament delivery device comprising a medicament dosage unit as hereinbefore described, In a most preferred embodiment the medicament. delivery device of the invention is an inhaler, e.g. a dry powder inhaler. Thus we especially provide an inhaler as hereinbefore described comprising a plurality of medicament dosage units.

Thus, according to a further feature of the invention we provide a dry powder inhaler comprising a medicament dosage unit as hereinbefore described. In a further embodiment we provide an inhaler as hereinbefore described comprising a plurality of medicament dosage units.

In the inhaler of the invention the medicament dosage units are preferably presented in a cartridge as hereinbefore described.

In a preferred embodiment a dry powder inhaler is provided with suitable indexing means, including a push button and a ratchet mechanism which engages with the upper surface of the container. Depression of the push button urges a push rod to push the container which is adjacent to the inhalation passage of the inhaler, downwards and almost fully out of the sleeve and into the inhalation passage.

A variety of medicaments may be administered by using the inhaler of the invention. Such medicaments are generally antibiotics, bronchodilators or other anti-asthma drugs. Such medicaments include, but are not limited to β₂-agonists, e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; mast cell stabilisers, e.g. sodium cromoglycate and ketotifen; bronchial anti-inflammatory agents, eg. nedocromil sodium; and steroids, e.g. beclomethasone dipropionate, fluticasone, budesonide and flunisolide.

Specific combinations of medicaments which may be mentioned include combinations of steroids, such as, beclomethasone dipropionate and formoterol; beclomethasone dipropionate and salmeterol; fluticasone and formoterol; fluticasone and salmeterol; budesonide and formoterol; budesonide and salmeterol; flunisolide and formoterol; and flunisolide and salmeterol. It is also within the scope of this invention to include combinations of one or more of the aforementioned steroids with one or more of the aforementioned β₂-agonists.

Further medicaments which may be mentioned include systemically active materials, such as, proteinaceous compounds and/or macromolecules, for example, hormones and mediators, such as insulin, human growth hormone, leuprolide and alpha interferon; growth factors, anticoagulants, immunomodulators, cytokines and nucleic acids.

The invention will now be described by way of example only and with reference to the accompanying drawings in which,
Figure 1 is a cross section of a dosage unit of the invention;
Figure 2 is a perspective view of a dosage unit;
Figure 3 is a perspective view of a dosage unit provided with a single central spacer and a sleeve;
Figure 4 is a perspective view of a dosage unit provided with a plurality of spacers and a sleeve and schematically represents the filling, sealing and emptying of the dosage unit;
Figure 5 is a cross-sectional schematic representation of a dosage unit filling process;
Figure 6 is a cross-sectional schematic representation of a cartridge containing dosage units of the invention;
Figure 7 is a cross-sectional schematic representation of a cartridge fitted in an inhaler, e.g. a TECHNOHALER;
Figure 8 is a perspective view of a dosage unit of the invention;
Figure 9 is a perspective cross-sectional view of a dosage unit;
Figure 10 is a perspective view of a sealed dosage unit;
Figure 11 is a perspective view of a dosage sealed unit in use;
Figure 12 is a schematic representation of a cartridge comprising dosage units of the invention;
Figure 13 is a schematic representation of a cartridge of the invention illustrating the emptying process.

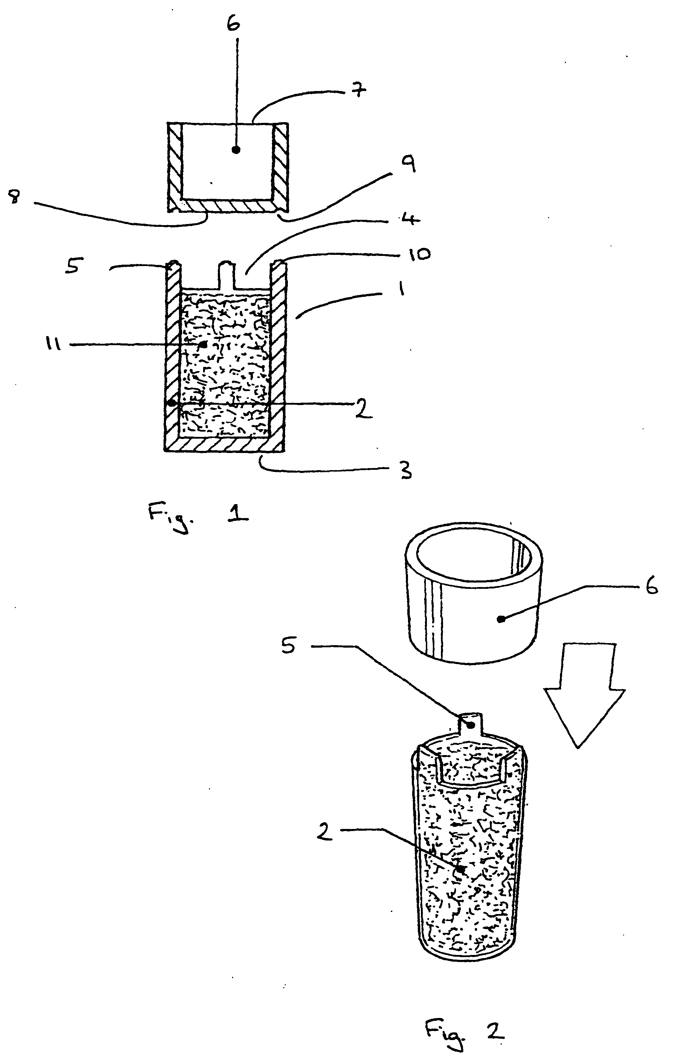

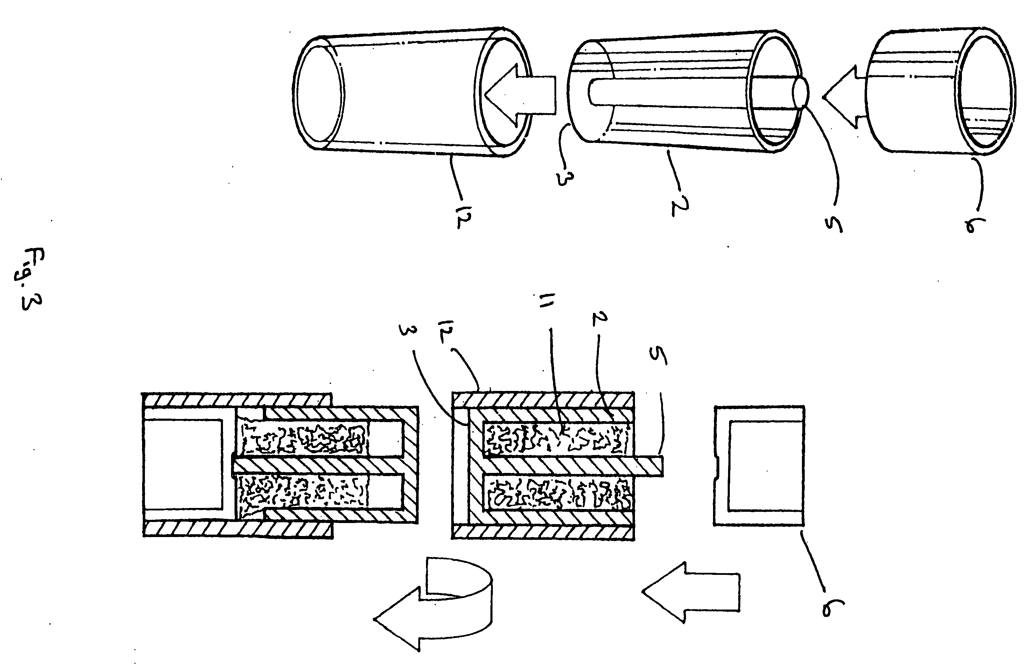

With reference to figures 1 and 2, a medicament dosage unit (1) comprises a container (2) provided with a closed end (3) and an open end (4). The periphery of the open end (4) is provided with spacers (5). A cap (6) is also shown which comprises a plug with an open end (7) and a closed end (8). In use the closed end (8) of the cap (6) is adjacent to the open end (4) of the container (2). The closed end (8) of the cap (6) is provided with peripheral grooves (9) adapted to engage with the spacers (5), which are provided with a ridge (10) to facilitate engagement.

The container (2) is filled with medicament (11).

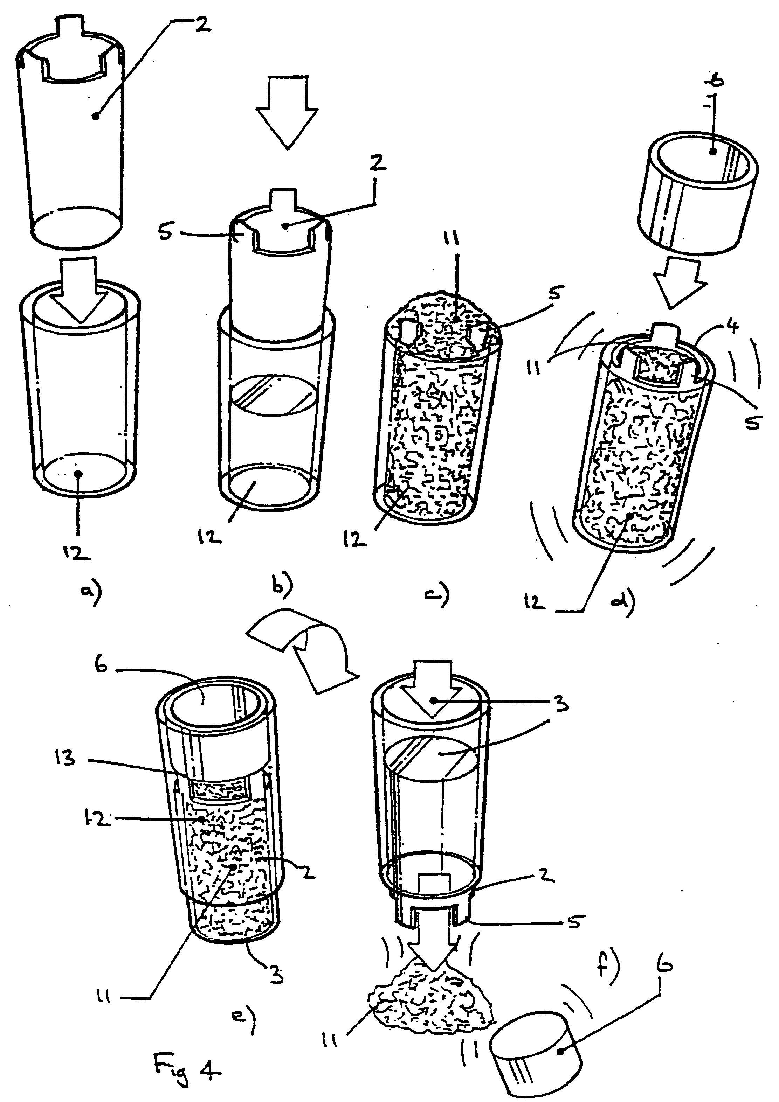

Referring to figures 3 and 4, a container (2) slidably engages with a sleeve (12) and is pushed so that only the spacers (5) protrude from the end of the sleeve (12). In figure 3 the single, central spacer protrudes from the sleeve, whereas in figure 4, all three spacers protrude from the sleeve. The container (2) may then be filled with medicament (11) and any excess medicament removed, for example, by agitating or shaking the cartridge, the container may then be closed with a cap (6) which is pushed onto the open end (4) of the container (2) such that the container (2) and the cap (6) slide into the sleeve (12). The closed end (3) of the container (2) protrudes from the sleeve (12) and the sleeve (12) forms a sealing engagement against the joint (13) between the cap (6) and container (2).

In figure 4 the unit may be inverted and a push rod (not shown) urges against the closed end (3) of the container (2) causing it to slide through the sleeve (12) such that the joint (13) protrudes from the sleeve (12) and the cap (6) falls away with the medicament (11) emptying from the container (2).

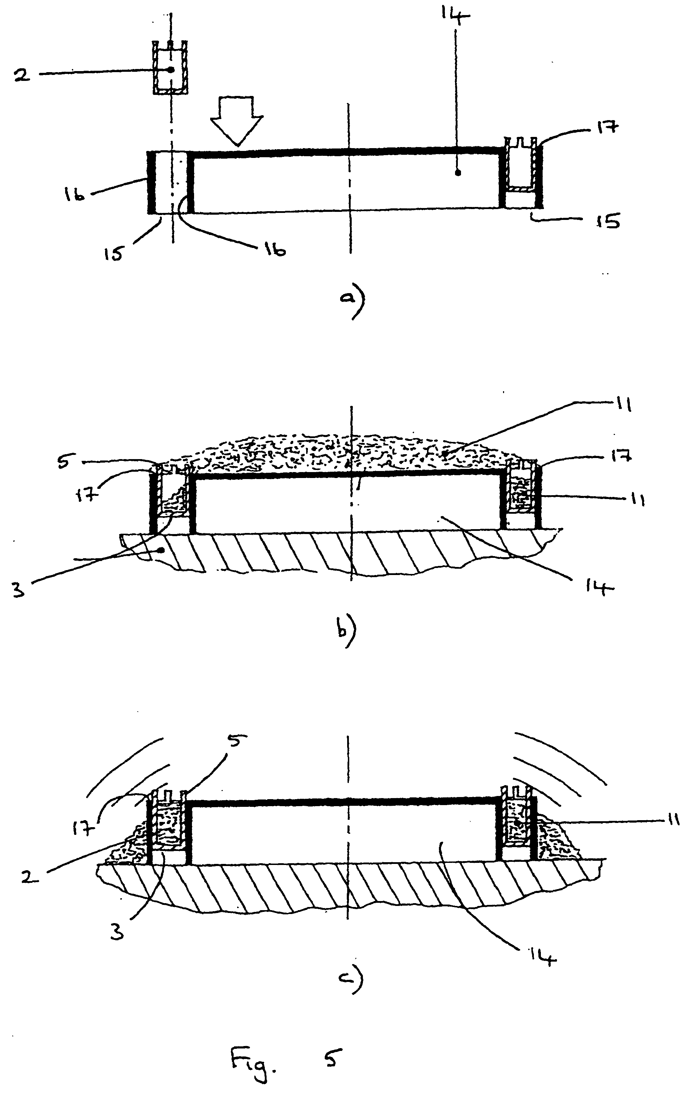

With reference to figure 5, a cartridge (14) is provided with apertures (15) to receive containers (2). The walls (16) of the apertures (15) form a sleeve (12) around the container (2). The containers (2) are pushed into the sleeves (12) so that the spacers (5) protrude and the open end (4) of the container abuts the periphery (17) of the sleeve (12). Again, medicament (11) may then be poured over the cartridge (14) and any excess medicament removed, for example, by agitating or shaking the cartridge.

With reference to figure 6, the containers (2) are sealed with a cap (6) and the unit is urged into the sleeve (12) so that a seal is formed at the joint (13) between the container (2) and the cap (6).

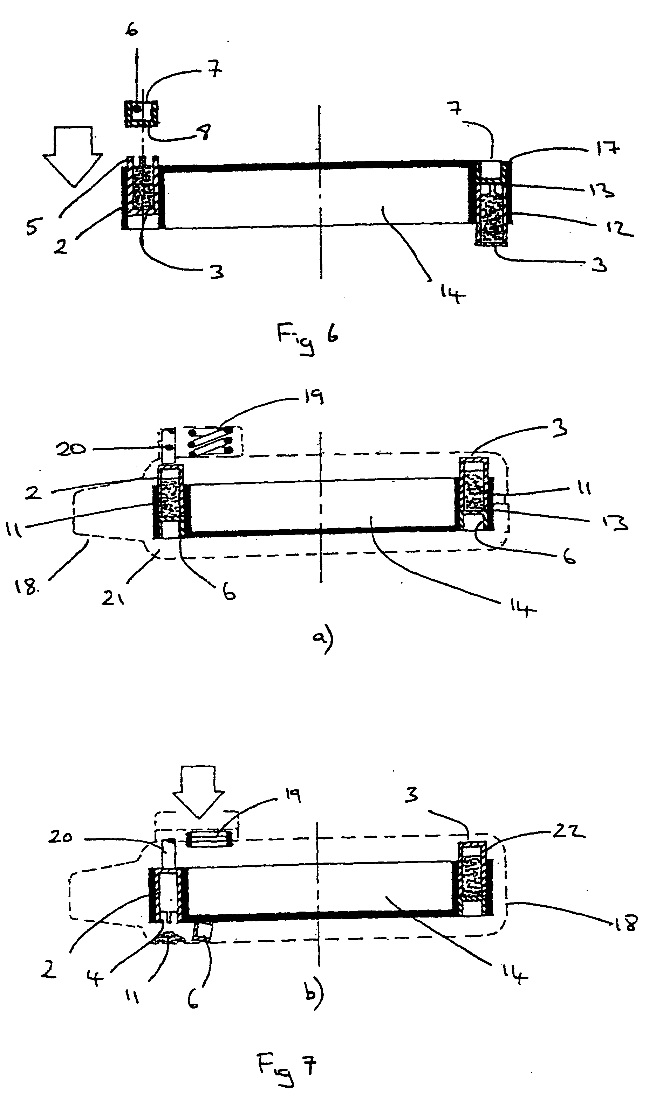

Referring to figure 7, in use the cartridge (14) is inverted and is placed in a dry powder inhaler (18). The inhaler is provided with a push rod (20) which may be biased with a spring (19).

The push rod (20) pushes against the closed end (3) of the container (2) urging it through the sleeve (12) until the joint (13) protrudes the sleeve (12). The cap (6) falls away. and medicament (11) empties into the inhalation passage (21). The cartridge (14) may optionally be rotatably mounted so that a second container (22) can be brought into contact with the push rod (20).

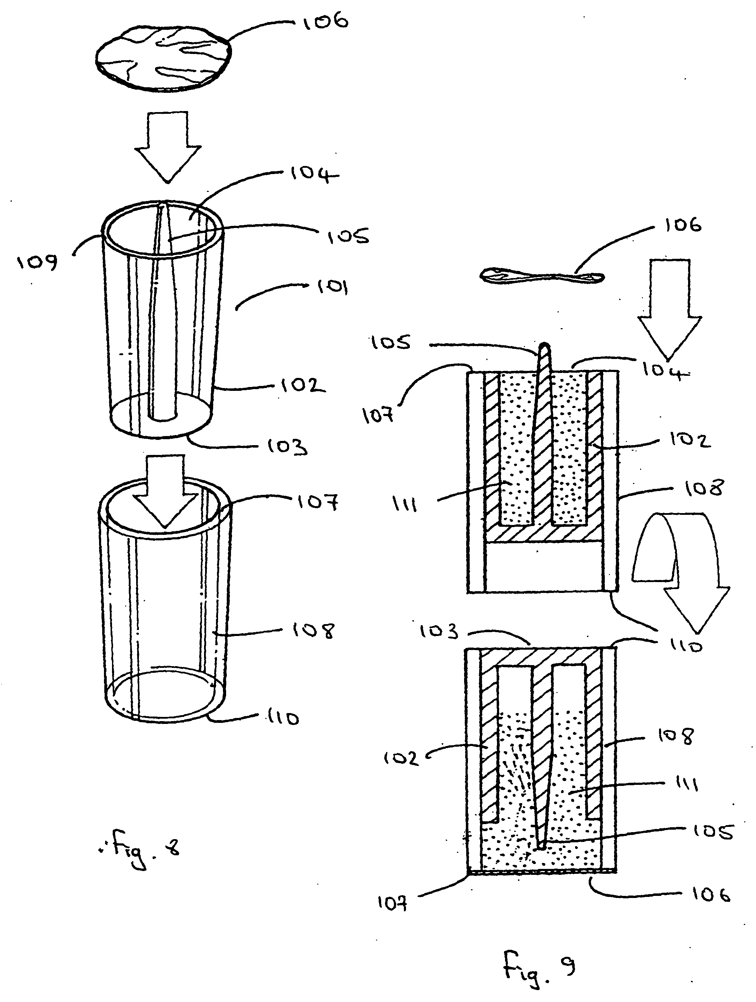

With reference to figures 8 and 9, a medicament dosage unit (101) comprises a container (102) provided with a closed end (103) and an open end (104). The container (102) is provided with a spacer (105) which protrudes beyond the open end (104). The container (102) is provided with a sleeve (108). The sleeve (108) is also provided with a frangible cover (106) which is adapted to overlie the peripheral edge (107) of the sleeve (108) which is adjacent the open end (104) of the container (102). In use, the container (102) slots into the sleeve (108) and is positioned such that the rim (109) of the container (102) is aligned with the peripheral edge (107) of the sleeve (108).

The container (102) is filled with medicament (111) prior to application of the cover (106). The spacer (105) prevents the medicament (111) from coming into contact with the cover (106) when the cover (106) is applied.

The container (102) is then urged into the sleeve (108) so that the closed end (103) is aligned with the peripheral edge (110) of the sleeve (108). The spacer (105) no longer protrudes from the sleeve (108) and the cover (106) is applied to the sleeve (108), being fixed to the peripheral edge (110).

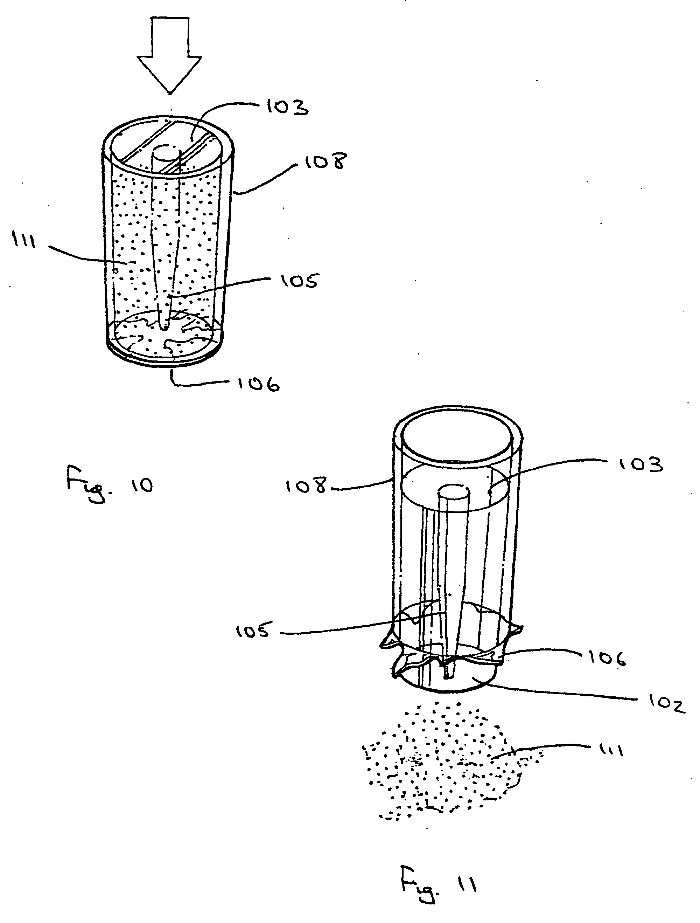

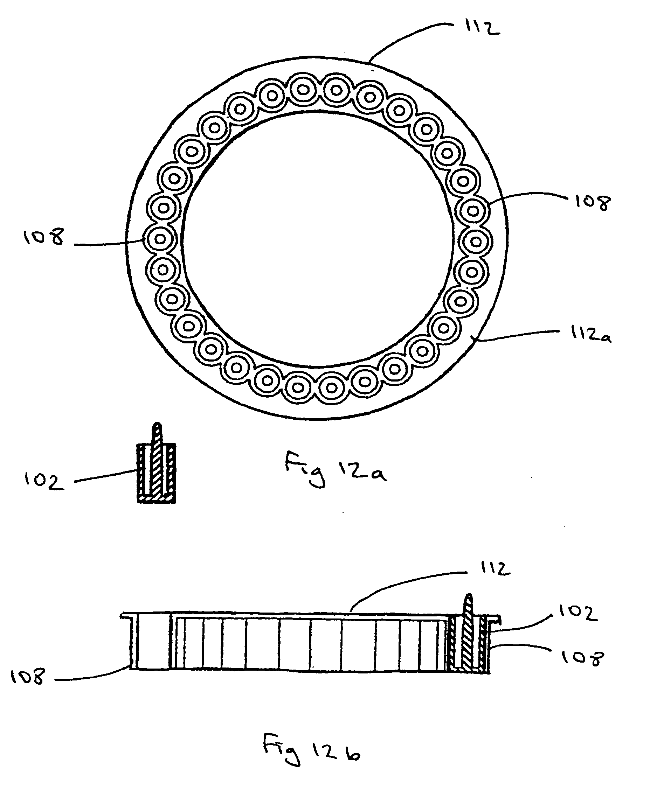

Referring to figures 10 and 11, the container (102) is slidably mounted within a sleeve (108). The container is inverted so that the closed end (103) of the container (102) is uppermost. When the container (102) is urged towards the frangible cover (106) e.g. by a push rod (not shown) the spacer (105) also acts as a means for breaking the cover (106) allowing the medicament (111) to empty.

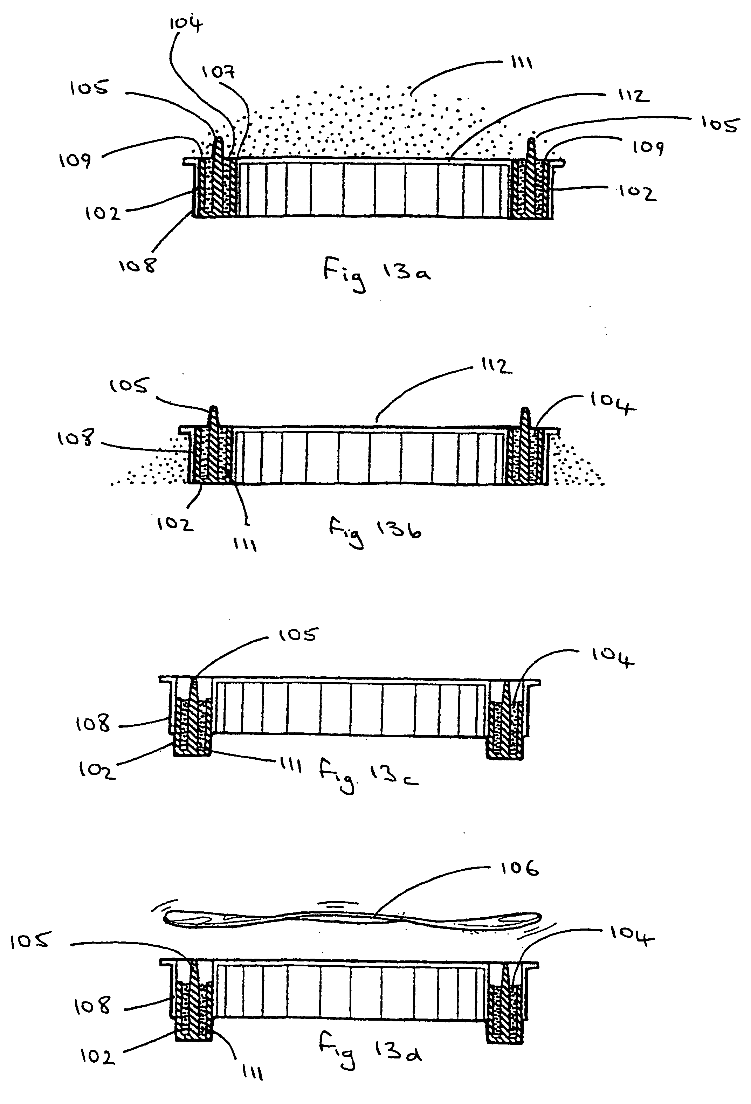

Referring to Figures 12a and 12b, a cartridge (112) comprises a plurality of sleeves (108) joined together. In each sleeve (108) is provided a medicament container (102). The cartridge (112) comprises a substantially circular array of sleeves (108) to enable it to be rotatable about a central point, bringing the container (102) in alignment with the inhalation chamber of an inhaler (not shown).

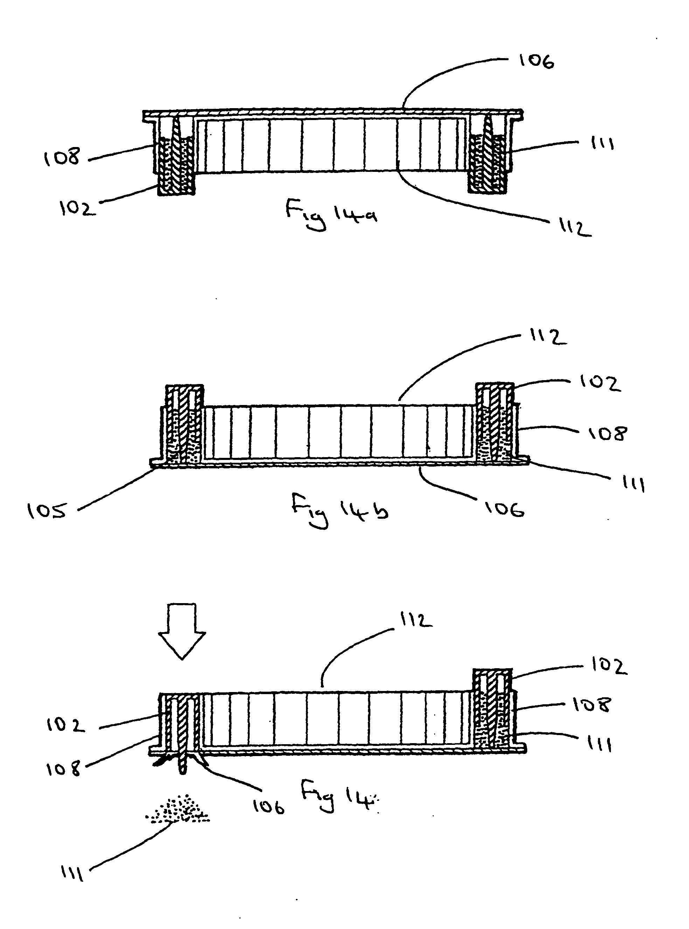

Referring to Figures 13a-d. The containers (102) are aligned in a cartridge (112) so that the rim (1) of the container is aligned with the peripheral edge (107) of the sleeve (108). The cartridge assembly (112) is then filled with medicament (111) so as to fill the containers (102). Any conventionally known filling method may be used, for example, the cartridge may be flooded with medicament and any excess removed or a more focussed filling method may be used. The cartridge may then optionally be placed on a vibrating platform so that any surplus medicament (111) is removed. The containers (102) are then slidably urged through the sleeve (108) so that the spacer (105) no longer protrudes from the open end (104) of the container (102). A foil seal (106) is then fixed to the cartridge over the open ends (104) of the containers (102).

Referring to Figures 14a-c, the cartridge (112) is filled with medicament (111) and sealed with a foil seal (106). In use, the cartridge is inverted and a push rod (not shown) urges the spacer (105) to break the frangible cover (106) allowing medicament (111) to empty by gravity.

## Claims

1. A medicament dosage unit (1) comprising a sleeve (12) and a medicament holding chamber (2), the sleeve (12) and the chamber (2) being dimensioned such that the sleeve (12) extends beyond the length of the chamber (2) and the unit being provided with an openable closure member (6) **characterised in that** the holding chamber (2) is adapted to form a slidable fit within the sleeve (12).

2. A medicament dosage unit (1) according to claim 1 **characterised in that** the dimensions are such that the chamber (2) will be filled to provide a single desired dose.

3. A medicament dosage unit (1) according to claim 1 **characterised in that** the chamber (2) is a substantially elongate member with an open end (4) and a closed end (3).

4. A medicament dosage unit (1) according to claim 1 **characterised in that** the chamber (2) is a cylindrical member.

5. A medicament dosage unit (1) according to claim 1 **characterised in that** the closure member (6) is a removable cap.

6. A medicament dosage unit (1) according to claim 5 **characterised in that** the cap rests on the sleeve (12).

7. A medicament dosage unit (1) according to claim 1 **characterised in that** the chamber (2) is provided with one or more spacers (5) at its open end (4).

8. A medicament dosage unit (1) according to claim 7 **characterised in that** at least two spacers (5) are present.

9. A medicament dosage unit (1) according to claim 7 **characterised in that** the spacers (5) are provided with a ridge (10) upon which the cap (6) may rest.

10. A medicament dosage unit (1) according to claim 5 **characterised in that** the cap (6) is substantially the same diameter as the chamber (2).

11. A medicament dosage unit (1) according to claim 5 **characterised in that** the cap (6) comprises a flat disc, a plug or an inverted cup.

12. A medicament dosage unit (1) according to claim 5 or 7 **characterised in that** the cap (6) provides a closed face abutting the chamber (2) and/or spacers (5).

13. A medicament dosage unit (1) according to claim 5 **characterised** that the length of the cap (6) is small relative to the length of the chamber (2).

14. A medicament dosage unit (1) according to claim 5 **characterised in that** the sleeve (12) is adapted to form a snug fit at least around the joint (13) formed between the open end (4) of the chamber (2) and the cap (6).

15. A medicament dosage unit (1) according to claim 14 **characterised in that** the sleeve (12) wraps around the whole of the circumference of the joint (13) so as to form a seal.

16. A medicament dosage unit (1) according to claim 1 **characterised in that** the sleeve (12) also acts as a support for the chamber (2).

17. A medicament dosage unit (1) according to claim 1 wherein the openable closure member (6) comprises a thin film or foil **characterised in that** the length of the sleeve (12) be substantially the same as the length of the chamber (2).

18. A medicament dosage unit (1) according to claim 7 or 17 **characterised in that** the length of the sleeve (12) is such that it forms a snug fit with the chamber (2) with only the spacers (5) protruding from the sleeve (12).

19. A medicament dosage unit (1) according to claim 1 **characterised in that** the closure member comprises a cover (106).

20. A medicament dosage unit (1) according to claim 19 **characterised in that** the cover (106) is fixed to the sleeve (12).

21. A medicament dosage unit (1) according to claim 1 **characterised in that** the cover (106) is substantially the same diameter as the sleeve (12).

22. A medicament dosage unit (1) according to claim 21 **characterised in that** the cover (106) comprises a frangible material.

23. A medicament dosage unit (1) according to claim 21 **characterised in that** the cover (106) comprises a material which is impermeable to moisture and/or is a moisture resistant.

24. A medicament dosage cartridge (14) comprising a plurality of dosage units (1) connected together, wherein each dosage unit (1) comprises a sleeve (12) and a medicament holding chamber (2), a sleeve (12) and the chamber (2) being dimensioned such that the sleeve (12) extends beyond the length of the chamber (2) and the unit being provided with an openable closure member (6) wherein the holding chamber (2) is adapted for a slidable fit within the sleeve (12).

25. A medicament dosage cartridge (14) according to claim 24 **characterised in that** the cartridge (14) comprises a plurality of sleeves (12) arranged around its periphery.

26. A medicament dosage cartridge (14) according to claim 25 **characterised in that** the sleeves (12) are connected together.

27. A medicament dosage cartridge (14) according to claim 26 **characterised in that** the units (1) are permanently attached to one another.

28. A medicament delivery device comprising a medicament dosage unit (1) which comprises a sleeve (12) and a medicament holding chamber (2), the sleeve (12) and the chamber (2) being dimensioned such that the sleeve (12) extends beyond the length of the chamber (2) and the unit being provided with an openable closure member (6) wherein the holding chamber (2) is adapted for a slidable fit within the sleeve (12).

29. A medicament delivery device according to claim 28 **characterised in that** the delivery device is an inhaler.

30. A medicament delivery device according to claim 29 **characterised in that** the inhaler is a dry powder inhaler.

31. A medicament delivery device according to claim 30 **characterised in that** the inhaler is provided with a plurality of medicament dosage units (1) in the form of a cartridge (14).

32. A medicament delivery device according to claim 31 **characterised in that** the device is provided with suitable indexing means.

## Patentansprüche

1. Eine Medikamenten-Dosierungseinheit (1), die eine Hülse (12) und eine Kammer (2) zur Aufnahme von Medikamenten umfasst, wobei die Hülse (12) und die Kammer (2) so dimensioniert sind, dass die Hülse (12) über die Länge der Kammer (2) hinausgeht und die Einheit mit einem beweglichen Verschlusselement (6) ausgestattet ist, **dadurch gekennzeichnet, dass** die Aufnahmekammer (2) angepasst ist, um eine gleitbare Einpassung in der Hülse (12) zu bilden.

2. Eine Medikamenten-Dosierungseinheit (1) nach Anspruch 1, **dadurch gekennzeichnet, dass** die Dimensionen so sind, dass die Kammer (2) gefüllt wird, um eine einfache gewünschte Dosis abzugeben.

3. Eine Medikamenten-Dosierungseinheit (1) nach Anspruch 1, **dadurch gekennzeichnet, dass** die Kammer (2) ein im Wesentlichen längliches Glied mit einem offenen Ende (4) und einem verschlossenen Ende (3) ist.

4. Eine Medikamenten-Dosierungseinheit (1) nach Anspruch 1, **dadurch gekennzeichnet, dass** die Kammer (2) ein zylinderförmiges Glied ist.

5. Eine Medikamenten-Dosierungseinheit (1) nach Anspruch 1, **dadurch gekennzeichnet, dass** das Verschlusselement (6) ein abnehmbarer Aufsatz ist.

6. Eine Medikamenten-Dosierungseinheit (1) nach Anspruch 5, **dadurch gekennzeichnet, dass** der Aufsatz auf der Hülse (12) aufliegt.

7. Eine Medikamenten-Dosierungseinheit (1) nach Anspruch 1, **dadurch gekennzeichnet, dass** die Kammer (2) mit einem oder mehreren Abstandshaltern (5) an ihrem offenen Ende (4) ausgestattet ist.

8. Eine Medikamenten-Dosierungseinheit (1) nach Anspruch 7, **dadurch gekennzeichnet, dass** mindestens zwei Abstandshalter (5) vorhanden sind.

9. Eine Medikamenten-Dosierungseinheit (1) nach Anspruch 7, **dadurch gekennzeichnet, dass** die Abstandshalter (5) mit einem Wulst (10) ausgestattet sind, auf dem der Aufsatz (6) aufliegen kann.

10. Eine Medikamenten-Dosierungseinheit (1) nach Anspruch 5, **dadurch gekennzeichnet, dass** der Aufsatz (6) im Wesentlichen den gleichen Durchmesser wie die Kammer (2) besitzt.

11. Eine Medikamenten-Dosierungseinheit (1) nach Anspruch 5, **dadurch gekennzeichnet, dass** der Aufsatz (6) eine flache Scheibe, einen Stopfen oder eine umgekehrte Kalotte umfasst.

12. Eine Medikamenten-Dosierungseinheit (1) nach Anspruch 5 oder 7, **dadurch gekennzeichnet, dass** der Aufsatz (6) eine geschlossene Fläche vorsieht, die an die Kammer (2) bzw. an die Abstandshalter (5) angrenzt.

13. Eine Medikamenten-Dosierungseinheit (1) nach Anspruch 5, **dadurch gekennzeichnet, dass** die Länge des Aufsatzes (6) im Verhältnis zu der Länge der Kammer (2) gering ist.

14. Eine Medikamenten-Dosierungseinheit (1) nach Anspruch 5, **dadurch gekennzeichnet, dass** die Hülse (12) angepasst ist, eine Schlichtpassung zu bilden, zumindest um die Fuge (13) herum, die zwischen dem offenen Ende (4) der Kammer (2) und dem Aufsatz (6) gebildet ist.

15. Eine Medikamenten-Dosierungseinheit (1) nach Anspruch 14, **dadurch gekennzeichnet, dass** sich die Hülse (12) um den gesamten Umfang der Fuge (13) herumlegt, so dass ein dichter Verschluss gebildet wird.

16. Eine Medikamenten-Dosierungseinheit (1) nach Anspruch 1, **dadurch gekennzeichnet, dass** die Hülse (12) auch als Halter für die Kammer (2) dient.

17. Eine Medikamenten-Dosierungseinheit (1) nach Anspruch 1, wobei das bewegliche Verschlusselement (6) einen dünnen Film oder eine Folie umfasst, **dadurch gekennzeichnet, dass** die Länge der Hülse (12) im Wesentlichen die gleiche Länge wie die der Kammer (2) besitzt.

18. Eine Medikamenten-Dosierungseinheit (1) nach Anspruch 7 oder 17, **dadurch gekennzeichnet, dass** die Länge der Hülse (12) so gestaltet ist, dass sie mit der Kammer (2) eine Schlichtpassung bildet, wobei nur die Abstandshalter (5) von der Hülse (12) abstehen.

19. Eine Medikamenten-Dosierungseinheit (1) nach Anspruch 1, **dadurch gekennzeichnet, dass** das Verschlusselement eine Abdeckung (106) umfasst.

20. Eine Medikamenten-Dosierungseinheit (1) nach Anspruch 19, **dadurch gekennzeichnet, dass** die Abdeckung (106) an der Hülse (12) befestigt ist.

21. Eine Medikamenten-Dosierungseinheit (1) nach Anspruch 1, **dadurch gekennzeichnet, dass** die Abdeckung (106) im Wesentlichen den gleichen Durchmesser wie die Hülse (12) besitzt.

22. Eine Medikamenten-Dosierungseinheit (1) nach Anspruch 21, **dadurch gekennzeichnet, dass** die Abdeckung (106) ein zerbrechliches Material umfasst.

23. Eine Medikamenten-Dosierungseinheit (1) nach Anspruch 21, **dadurch gekennzeichnet, dass** die Abdeckung (106) ein Material umfasst, das feuchtigkeitsundurchlässig bzw. feuchtigkeitsbeständig ist.

24. Eine Medikamenten-Dosierungs-Cartridge (14), die eine Vielzahl von miteinander verbundenen Dosierungseinheiten (1) umfasst, wobei jede Dosierungseinheit (1) eine Hülse (12) und eine Kammer (2) zur Aufnahme von Medikamenten umfasst, wobei eine Hülse (12) und die Kammer (2) so dimensioniert sind, dass die Hülse (12) über die Länge der Kammer (2) hinausgeht und die Einheit mit einem beweglichen Verschlusselement (6) ausgestattet ist, wobei die Aufnahmekammer (2) für eine gleitbare Einpassung in der Hülse (12) angepasst ist.

25. Eine Medikamenten-Dosierungs-Cartridge (14) nach Anspruch 24, **dadurch gekennzeichnet, dass** die Cartridge (14) eine Vielzahl von Hülsen (12) umfasst, die um ihre Peripherie herum angeordnet sind.

26. Eine Medikamenten-Dosierungs-Cartridge (14) nach Anspruch 25, **dadurch gekennzeichnet, dass** die Hülsen (12) miteinander verbunden sind.

27. Eine Medikamenten-Dosierungs-Cartridge (14) nach Anspruch 26, **dadurch gekennzeichnet, dass** die Einheiten (1) dauerhaft aneinander befestigt sind.

28. Eine Vorrichtung zur Abgabe von Medikamenten, die eine Medikamenten-Dosierungseinheit (1) umfasst, die eine Hülse (12) und eine Kammer (2) zur Aufnahme von Medikamenten umfasst, wobei die Hülse (12) und die Kammer (2) so dimensioniert sind, dass die Hülse (12) über die Länge der Kammer (2) hinausgeht und die Einheit mit einem beweglichen Verschlusselement (6) ausgestattet ist, wobei die Aufnahmekammer (2) für eine gleitbare Einpassung in der Hülse (12) angepasst ist.

29. Eine Vorrichtung zur Abgabe von Medikamenten nach Anspruch 28, **dadurch gekennzeichnet, dass** die Abgabevorrichtung ein Inhalator ist.

30. Eine Vorrichtung zur Abgabe von Medikamenten nach Anspruch 29, **dadurch gekennzeichnet, dass** der Inhalator ein Trockenpulver-Inhalator ist.

31. Eine Vorrichtung zur Abgabe von Medikamenten nach Anspruch 30, **dadurch gekennzeichnet, dass** der Inhalator mit einer Vielzahl von Medikamenten-Dosierungseinheiten (1) in der Form einer Cartridge (14) ausgestattet ist.

32. Eine Vorrichtung zur Abgabe von Medikamenten nach Anspruch 31, **dadurch gekennzeichnet, dass** die Vorrichtung mit einem geeigneten Anzeigemittel ausgestattet ist.

## Revendications

1. Une unité (1) de dosage de médicament qui comprend un manchon (12) et une chambre (2) de rétention de médicament, le manchon (12) et la chambre (2) étant dimensionnés de telle sorte que le manchon (12) se prolonge au-delà de la longueur de la chambre (2), cette unité étant pourvue d'un élément (6) de fermeture pouvant être ouvert, **caractérisée en ce que** la chambre (2) de rétention est adaptée de façon à former un emboîtement coulissant à l'intérieur du manchon (12).

2. Une unité (1) de dosage de médicament selon la revendication 1, **caractérisée en ce que** ses dimensions sont telles que la chambre (2) se remplira pour fournir une dose unique souhaitée.

3. Une unité (1) de dosage de médicament selon la revendication 1, **caractérisée en ce que** la chambre (2) est un élément substantiellement allongé avec une extrémité (4) ouverte et une extrémité (3) fermée.

4. Une unité (1) de dosage de médicament selon la revendication 1, **caractérisée en ce que** la chambre (2) est un élément cylindrique.

5. Une unité (1) de dosage de médicament selon la revendication 1, **caractérisée en ce que** l'élément (6) de fermeture est un couvercle amovible.

6. Une unité (1) de dosage de médicament selon la revendication 5, **caractérisée en ce que** le couvercle s'appuie sur le manchon (12).

7. Une unité (1) de dosage de médicament selon la revendication 1, **caractérisée en ce que** la chambre (2) est pourvue d'un ou plusieurs entretoises (5) à son extrémité (4) ouverte.

8. Une unité (1) de dosage de médicament selon la revendication 7, **caractérisée en ce qu'**elle comporte au moins deux entretoises (5).

9. Une unité (1) de dosage de médicament selon la revendication 7, **caractérisée en ce que** les entretoises (5) sont pourvues d'un élément en saillie (10) sur lequel peut s'appuyer le couvercle (6).

10. Une unité (1) de dosage de médicament selon la revendication 5, **caractérisée en ce que** le couvercle (6) a substantiellement le même diamètre que la chambre (2).

11. Une unité (1) de dosage de médicament selon la revendication 5, **caractérisée en ce que** le couvercle (6) comprend un disque plat, un bouchon ou une calotte inversée.

12. Une unité (1) de dosage de médicament selon la revendication 5 ou 7, **caractérisée en ce que** le couvercle (6) constitue une superficie fermée qui touche les bords de la chambre (2) et/ou des entretoises (5).

13. Une unité (1) de dosage de médicament selon la revendication 5, **caractérisée en ce que** la longueur du couvercle (6) est petite par rapport à la longueur de la chambre (2).

14. Une unité (1) de dosage de médicament selon la revendication 5, **caractérisée en ce que** le manchon (12) est adapté de façon à former un emboîtement serré au moins autour du joint (13) formé entre l'extrémité (4) ouverte de la chambre (2) et le couvercle (6).

15. Une unité (1) de dosage de médicament selon la revendication 14, **caractérisée en ce que** le manchon (12) enveloppe la totalité de la circonférence du joint (13) de façon à former une fermeture hermétique.

16. Une unité (1) de dosage de médicament selon la revendication 1, **caractérisée en ce que** le manchon (12) agit également comme support pour la chambre (2).

17. Une unité (1) de dosage de médicament selon la revendication 1 dans laquelle l'élément (6) de fermeture pouvant être ouvert comprend une pellicule ou une plaque fine, **caractérisée en ce que** la longueur du manchon (12) est substantiellement la même que la longueur de la chambre (2).

18. Une unité (1) de dosage de médicament selon la revendication 7 ou 17, **caractérisée en ce que** la longueur du manchon (12) est telle qu'il forme un emboîtement serré avec la chambre (2), et seuls les entretoises (5) dépassent du manchon (12).

19. Une unité (1) de dosage de médicament selon la revendication 1, **caractérisée en ce que** l'élément (6) de fermeture comprend une couverture (106).

20. Une unité (1) de dosage de médicament selon la revendication 19, **caractérisée en ce que** la couverture (106) est fixée au manchon (12).

21. Une unité (1) de dosage de médicament selon la revendication 1, **caractérisée en ce que** la couverture (106) a substantiellement le même diamètre que le manchon (12).

22. Une unité (1) de dosage de médicament selon la revendication 21, **caractérisée en ce que** la couverture (106) comprend un matériau fragile.

23. Une unité (1) de dosage de médicament selon la revendication 21, **caractérisée en ce que** la couverture (106) comprend un matériau qui est imperméable à l'humidité et/ou est résistant à l'humidité.

24. Une cartouche (14) de dosage de médicament qui comprend une pluralité d'unités (1) de dosage reliées les unes aux autres, dans laquelle chaque unité (1) de dosage comprend un manchon (12) et une chambre (2) de rétention de médicament, un manchon (12) et la chambre (2) étant dimensionnés de telle sorte que le manchon (12) se prolonge au-delà de la longueur de la chambre (2), l'unité étant pourvue d'un élément (6) de fermeture pouvant être ouvert, dans lequel la chambre (2) de rétention est adaptée de façon à former un emboîtement coulissant à l'intérieur du manchon (12).

25. Une cartouche (14) de dosage de médicament selon la revendication 24, **caractérisée en ce que** la cartouche (14) comprend une pluralité de manchons (12) disposés autour de sa périphérie.

26. Une cartouche (14) de dosage de médicament selon la revendication 25, **caractérisée en ce que** les manchons (12) sont reliés entre eux.

27. Une cartouche (14) de dosage de médicament selon la revendication 26, **caractérisée en ce que** les unités (1) sont unies entre elles de façon permanente.

28. Un dispositif d'administration de médicament qui comprend une unité (1) de dosage de médicament qui comprend un manchon (12) et une chambre (2) de rétention de médicament, le manchon (12) et la chambre (2) étant dimensionnés de telle sorte que le manchon (12) se prolonge au-delà de la longueur de la chambre (2), l'unité étant pourvue d'un élément (6) de fermeture pouvant être ouvert, dans lequel la chambre (2) de rétention est adaptée de façon à former un emboîtement coulissant à l'intérieur du manchon (12).

29. Un dispositif d'administration de médicament selon la revendication 28, **caractérisé en ce que** le dispositif d'administration est un inhalateur.

30. Un dispositif d'administration de médicament selon la revendication 29, **caractérisé en ce que** l'inhalateur est un inhalateur de poudre sèche.

31. Un dispositif d'administration de médicament selon la revendication 30, **caractérisé en ce que** l'inhalateur est pourvu d'une pluralité d'unités (1) de dosage de médicament sous forme d'une cartouche (14).

32. Un dispositif d'administration de médicament selon la revendication 31, **caractérisé en ce que** le dispositif est pourvu des moyens diviseurs adéquats.
